# EUROPEAN PATENT SPECIFICATION

(11) **EP 3 459 577 B1**
(45) Date of publication and mention of the grant of the patent: **04.11.2020**
(21) Application number: 17192585.2
(22) Date of filing: 22.09.2017
(51) Int. Cl.: A61M 11/06, B05B 1/26, B05B 7/00, B05B 7/24, A61M 15/00, A61M 16/06, A61M 16/20

(54) **NEBULIZER FOR AEROSOLIZING A DRUG SOLUTION WITH AN INNER FLARED-OUT STRUCTURE LIMITING THE AEROSOL WASTE**
VERNEBLER ZUR AEROSOLISIERUNG EINER MEDIKAMENTENLÖSUNG MIT EINER INNEREN, AUFGEBÖRDELTEN STRUKTUR ZUR BEGRENZUNG DES AEROSOLABFALLS
NÉBULISEUR POUR L'AÉROSOLISATION D'UNE SOLUTION MÉDICAMENTEUSE À STRUCTURE INTERNE ÉVASÉE LIMITANT LES DÉCHETS D'AÉROSOL

(43) Date of publication of application: 27.03.2019
(73) Proprietor: Air Liquide Medical Systems S.r.l., 20148 Milano (IT)
(72) Inventor: ALBERICI, Luca, 25030 RONCADELLE (BS) (IT); SANDONI, Giuseppe, 25124 BRESCIA (IT); RUOCCO, Alessandra, 25030 ORZIVECCHI (IT)
(74) Representative: Pittis, Olivier

(56) References cited:
- EP-A1- 0 170 715
- EP-A1- 1 541 186
- WO-A2-97/29799
- WO-A2-2011/073756
- US-A1- 2001 013 341
- US-A1- 2012 174 917
- US-B1- 7 581 718

## Description

The present invention concerns a nebulizer comprising a tubular element with a flared-out portion useable for aerosoltherapy.

Nebulizing devices, commonly called nebulizers, are used for delivering inhalation therapies, i.e. aerosoltherapies, to patients in need thereof. They are used in combination with a source of gas, typically compressed air, for nebulizing a drug solution thereby obtaining a mist containing droplets having suitable sizes allowing them to reach the lower airways of the patients that have to inhale said mist in the course of their aerosoltherapies.

Different structures of nebulizers according to the prior art are taught by EP-A-1541186, WO-A-97/29799, EP-A-170715 and WO-A-2011/073756.

Document US 2012/0174917 A1 discloses a press-type medical nebulizer and a pressing means thereof. The nebulizer includes a medicament container, a medical spray generator, and a pressing means. The medicament container comprises a cylinder, a shroud and a filter sheet. The interior of the cylinder has a chamber penetrating the top of the cylinder. The outer surface of the cylinder is provided with a mixed gas exit. The periphery of the mixed gas exit protrudes to form an inhalation pipe in communication with the mixed gas exit. The shroud is inserted into an upper space of the chamber to seal the top of the cylinder. The shroud comprises a lower cap and a filter casing combined with the lower cap. The interior of the lower cap are independently formed with an intake channel and an exhaust channel. The intake channel is positioned to correspond to the chamber, and the exhaust channel is positioned to correspond to the mixed gas exit. The top surface of the lower cap is provided with an intake hole in communication with the intake channel and an exhaust hole in communication with the exhaust channel. The lower cap has an one-way intake valve disposed across the intake channel for selectively closing the intake hole, and an one-way exhaust valve disposed across the exhaust channel for selectively closing the exhaust hole. Further, a mixed gas channel is formed between the lower cap and the inner wall of the cylinder on the side of the mixed gas exit. The mixed gas channel is in communication with the chamber and the mixed gas exit. The medical spray generator is inserted inside the lower portion of the cylinder. The medical spray generator comprises a conical post, a conical shroud and a stopping block. The conical post is formed by punching the inner bottom wall of the cylinder toward the chamber. The recessed side of the conical post is formed with a pressurized gas channel. The top of the conical post is provided with a pressurized gas exit in communication with the chamber and the pressurized gas channel. The conical shroud has a through-hole. The conical shroud is disposed around the protruding side of the conical post, so that a gap is formed between the conical shroud and the conical post to form a flow channel. The stopping block is disposed above the conical shroud and the conical post to correspond to the pressurized gas exit and the opening of the flow channel. The operating principle of producing the medical spray by the nebulizer of US 2012/0174917 A1 comprises the following. First, a medicament is filled in the chamber. The medicament is prepared for treating the disease of a patient. The level of the medicament does not exceed the pressurized gas exit, and the medicament will flow into the flow channel. A pressurized gas flows from the pressurized gas channel into the pressurized gas exit of a smaller diameter, which generate a so-called "Venturi Effect". As a result, the pressurized gas draws the medicament out of the flow channel. The medicament is drawn by the pressurized gas, so that the medicament collides with the pressurized gas to become tiny particles. The tiny particles of the medicament is sprayed by the pressurized gas to collide with the stopping block, thereby atomizing the medicament. The atomized medicament is mixed with the pressurized gas to form a medical spray. The thus-formed medical spray is filled in the intake channel. The patient holds the inhalation pipe to inhale the medical spray from the intake channel through the chamber, the mixed gas channel, the mixed gas exit into his/her lungs. The inhalation of the patient forces the one-way intake valve to open the intake hole and forces the one-way exhaust valve to tightly close the exhaust hole. At this time, the external air enters the intake hole to compensate for the insufficient quantity of breath of the patient. Also, the external air guides most of the medical spray into the mixed gas exit, so that the patient can inhale sufficient amount of the medical spray without inhaling too much air. In this way, the efficiency in inhaling the medicament is increased, and the duration of the inhalation treatment is reduced. On the other hand, after the patient inhales the medical spray, the patient will subsequently exhale the air in his/her lungs. At this time, the exhalation of the patient forces the one-way exhaust valve to open the exhaust hole. The waste air exhaled by the patient is exhausted to the outside via the mixed gas exit and the exhaust channel, and then filtered by the filter sheet. In this way, the viruses and bacteria contained in the waste air exhaled by the patient can be filtered out without spreading to the outside to contaminate the surrounding environment. At this time, since the patient is exhaling rather than inhaling, the one-way intake valve closes the exhaust hole again, thereby preventing the medical spray from dispersing to the outside. Further, since the cross-sectional area of the mixed gas channel is much smaller than that of the exhaust channel, the waste air exhaled by the patient cannot enter the channel but can be only exhausted to the outside via the exhaust channel.

US 2001/0013341 A1 discloses an inhalation atomizer, the housing of which includes an atomizer housing lower part and an atomizer housing upper part. At the atomizer housing upper part there is arranged a mouthpiece through which the patient inhales the aerosol generated in the inhalation atomizer and into which the patient also exhales. The inhalation atomizer has a valve box cover which closes a valve box and has a first cover opening and a second cover opening which communicates the valve box with the surroundings. Through the first cover opening the air which the patient exhales into the mouthpiece during the expiration process flows out of the inhalation atomizer housing. Through the second cover opening ambient air flows into the inhalation atomizer housing during the inspiration process in which the patient inhales through the mouthpiece the aerosol generated in the inhalation atomizer. A partition wall divides the valve box in two sub-chambers. The first sub-chamber has a first valve box opening, which communicates the valve box with the interior of the inhalation atomizer housing. During expiration, the respiration air of the patient flows who exhales into the mouthpiece flows through said opening. For this reason the first valve box opening is arranged relatively near the mouthpiece of the inhalation atomizer. On the side facing the valve box the first valve box opening is formed as a valve seat so that a one-piece valve element can close the first valve box opening during the inspiration process. For this purpose the first valve box opening is for instance provided with a peripheral edge, which acts as a sealing lip. The valve element rests on the partition wall so that together with the edge a valve seat is realized. The second sub-chamber has a second valve box opening, which communicates the valve box with the interior of the inhalation atomizer housing so that during the inspiration process ambient air can flow into the housing of the inhalation atomizer. The side of the second cover opening facing the valve box is designed as a valve seat. This is for instance obtained by an edge acting as sealing lip when the valve element abuts against the edge during the expiration process. A first section of the valve element lies in the first sub-chamber of the valve box. Therein the first section is arranged such that the first section of the valve element rests on the valve seat of the first valve box opening during the inspiration process and closes the same. Thereby it is guaranteed that during the inspiration process the patient inhales the aerosol which is generated in the inhalation atomizer. During the expiration process the first section of the valve element is lifted off the valve seat and opens or releases the first valve box opening without closing the first cover opening. The air exhaled by the patient therefore can flow through the mouthpiece, the first valve box opening, the first sub-chamber and the first cover opening. A second section of the valve element lies in the second sub-chamber of the valve box. The second section of the valve element is arranged such that the second section abuts against the valve seat of the second cover opening of the valve box cover during the expiration process. Thereby, during the expiration process, the second section of the valve element closes the second cover opening so that the air exhaled by the patient into the mouthpiece does not cause the aerosol generated in the inhalation atomizer housing to get to the outside together with the exhaled air through the second valve box opening, the second sub-chamber and the second cover opening. During the inspiration process the second section of the valve element opens the second cover opening without closing the second valve box opening. Thus, during inspiration through the mouthpiece, the patient can inhale ambient air through the second cover opening, the second sub-chamber and the second valve box opening. Therein, the air thus inhaled takes along the aerosol generated in the inhalation atomizer housing and supplies it to the patient via the mouthpiece. The housing of the inhalation atomizer comprises a lower housing part and an upper housing part. At the upper housing part there is arranged the mouthpiece. Furthermore, the valve box is closed towards the outside by the valve box cover. The valve box comprises the first sub-chamber and the second sub-chamber which are separated from the housing interior by a wall. The valve box communicates with the housing interior via the valve box openings and towards the surroundings via the openings of the valve box cover. The openings are closed by the flexible valve element, which rests upon the partition wall and is secured via a snap-in projection as well as by the clamping effect exerted by the valve box cover.

In a nebulizer, a part of the drug solution is however lost on the inner walls of the device, i.e. by forming droplets. The quantity of solution thus wasted is variable but can be very important, i.e. typically of at least 1 cc (cubic centimeter = cm³).

This is a real problem because the drug solution droplets stuck on the walls cannot be nebulized and hence cannot be used for treating the patient. For instance, if a quantity of 3 cc of drug solution is required for treating a patient and introduced into the reservoir of the nebulizer, then wasting from between 1 to 1,5 cc of the drug solution on the walls negatively impacts the efficacy of the therapy as between 30% and 50% of the drug solution do not reach the patient's airways.

The goal of the present invention is to provide an improved nebulizer, wherein the quantity of drug solution wasted on the inner walls of the device is lowered/minimized, namely of less than 1 cc, preferably of less than 0,8 cc.

In other words, the problem to be solved is to improve the nebulizer architecture for limiting the waste of drug on the inner walls.

The solution of the invention is a nebulizer according to Claim 1. The dependent claims define preferred embodiments of the invention.

Depending on the embodiment, the nebulizer of the invention can comprise one or several of the following features:
- the flared-out portion is configured for condensing a part of the nebulization mist so that the condensed droplets thus formed fall back in the reservoir tank, i.e. those droplets cannot condense on the other inner parts of the nebulizer and the quantity of solution wasted is limited/minimized.
- the tubular element is detachably attached to the inner conduit by a first free extremity of the tubular element.
- the flared-out portion comprises an annular border.
- the tubular element comprises the flared-out portion at a second free extremity of the tubular element.
- the flared-out portion comprises an enlarged diameter of less than 3 cm, preferably of between 2 and 3 cm.
- the inner diameter of the hollow tubular element is greater than 1 cm and less than 2.5 cm, preferably of between about 1,5 and about 2 cm.
- the tubular element is co-axially arranged around the inner conduit.
- the hollow tubular element comprising the flared-out portion and the axially-arranged inner conduit are integrally arranged.
- the tubular element comprises, at the first end, one or several elongated parts, such as arms, projecting upwardly cooperating with one or several lodgings for securing the tubular element into the nebulizer body.
- the tubular element comprises two parallel elongated arms projecting upwardly.
- the tubular element comprising the flared-out portion is arranged so as to form a sleeve around the lower end of the inner conduit.
- the reservoir comprises:
   - a liquid tank for receiving the liquid to be aerosolized, and
   - an inner sleeve conduit projecting axially and upwardly into the liquid tank, said inner sleeve conduit forming a sleeve around the nozzle element of the aerosol-generating system, said inner sleeve conduit being separated from the nozzle element by a spacing, preferably at a distance of less than about 2 mm, and comprising an upper outlet facing the nozzle orifice of the nozzle element.
- the flared-out portion of the tubular element comprises an enlarged opening.
- the reservoir comprises a liquid tank having a cup-like shape or similar.
- the axially-arranged inner conduit is directed toward the deflector element.
- the deflector element is facing the nozzle orifice of the nozzle element and the upper outlet of the inner conduit.
- the deflector element is arranged into the axially-arranged inner conduit.
- the deflector element is arranged at the exit of the lower end of the axially-arranged inner conduit.
- the deflector element has a blade-shape or similar.
- the deflector element comprises a portion having a triangular or a trapezoidal cross-section structure.
- the inner sleeve conduit further comprises a lower inlet located close to the bottom of the liquid tank of the reservoir, preferably at a distance of less than about 2 mm.
- the nozzle element of the aerosol-generating system comprises an upstream connecting-portion for connecting a gas line or similar thereto that is used for feeding an under pressure-gas, such as air.
- it further comprises a respiratory interface in fluid communication with the nebulization chamber.
- the respiratory interface comprises a mouthpiece, a mouth mask or a facial mask.
- one or several parts of the nebulizer are made of polymer material(s).
- the tubular element is molded in one-piece and made of a plastic material.
- the deflector, the axially-arranged inner conduit and the inner sleeve conduit are made in one-piece, preferably by molding or the like of a plastic material or similar.

A preferred embodiment of a nebulizer according to the present invention is shown in the enclosed Figures, among which:
- Figure 1 represents a general view of an embodiment of a nebulizer according to the present invention,
- Figure 2 is a longitudinal sectional view of the nebulizer of Figure 1,
- Figure 3 is an enlarged view of a part of Figure 2, and
- Figures 4 and 5 are different views of the flared-out tubular element arranged in the nebulizer of Figure 1 and visible in Figures 2 and 3.

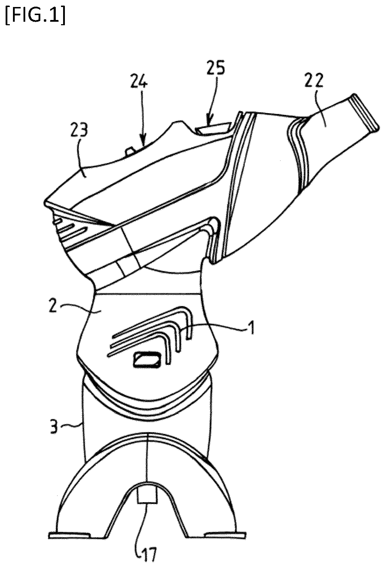

Figure 1 is a general view of a nebulizer 1 for aerosolizing a liquid according to the present invention.

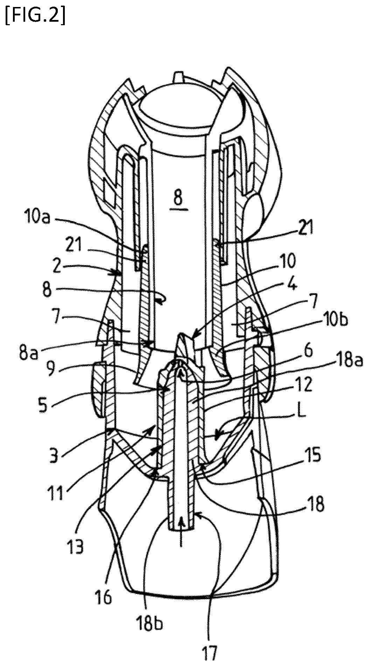

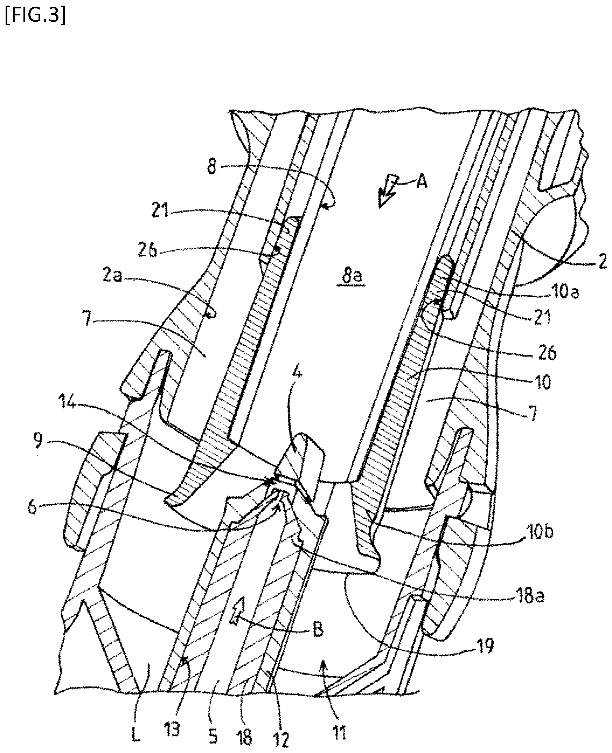

The nebulizer 1 comprises:
- a lower part forming a reservoir 3 for receiving a liquid L to be aerosolized, such as a drug-containing solution,
- a main part forming the nebulizer body 2 that comprises an aerosol-generating system 4, 5, 6 (cf. Fig. 2 & 3) for generating the aerosol and further a nebulization chamber 7 for recovering and containing the aerosol created by the aerosol-generating system 4, 5, 6, and
- a respiratory interface 22 comprising a mouthpiece, a mouth mask, a facial mask or similar for delivering the aerosol mist to the patient, said respiratory interface 22 being in fluid communication with the nebulization chamber 7. In Fig. 1, the respiratory interface 22 is a mouthpiece. The nebulizer body 2 is located between and fixed to the reservoir 3 and the respiratory interface 22.

Further, the nebulizer body 1 comprises a detachable cover 23 that comprises 2 valves 24, 25 for controlling air flows entering into or exiting the nebulizer 1, while the patient is breathing, i.e. inhaling or exhaling gas, as explained below.

As illustrated in Figures 2 and 3, the aerosol-generating system 4, 5, 6 comprises a nozzle element 5, such as a hollow gas conduit or similar, comprising an inner passage terminated by a nozzle orifice 6, for conveying a gas, such as air.

The aerosol-generating system 4, 5, 6 further comprises a deflector element 4, usually called 'deflector', facing the nozzle orifice 6 of the nozzle element 5 so that the gas delivered by the nozzle orifice 6 impacts the front face or part 4a of the deflector element 4.

The deflector 4 is, in the present embodiment, a blade-shape element comprising a trapezoidal or triangular cross section. Of course, it can have any other suitable shape.

As visible in Figures 2 and 3, the nebulizer body 2 comprises an axially-arranged inner conduit 8 comprising a lower end 8a. Said axially-arranged inner conduit 8 axially traverses the nebulizer body 2 and is directed toward the deflector 4 so that air inspired by the patient is convoyed by the axially-arranged inner conduit 8 and impacts the rear part 4b of the deflector 4.

Actually, the deflector 4 is positioned at the exit of the axially-arranged inner conduit 8, namely in the region of the lower end 8a of the axially-arranged inner conduit 8, so as to face the exit of the axially-arranged inner conduit 8. The blade-shape element forming the deflector 4 is preferably secured to the inner wall of the inner conduit 8 and diametrically-arranged into said inner conduit 8.

The nebulization chamber 5 is arranged around at least a part of said inner conduit 8 so as to recover and to contain at least a part of the nebulization mist created by the aerosol-generating system 4, 5, 6.

The reservoir 3 is designed for receiving and containing a liquid L to be aerosolized, in particular a drug-containing solution. It includes a liquid tank 11 have a cup-like shape for storing the liquid L.

The reservoir 3 comprises a liquid tank 11, also called liquid vessel or liquid compartment, for receiving the drug-solution L to be aerosolized, and is traversed by an axial tubular structure 18, such as a little conduit, projecting upwardly and downwardly. Said axial tubular structure 18 traverses the bottom 16 of the liquid tank 11 and is integral with said bottom 16. Preferably, they are made of one piece, for instance by molding or the like.

The upward portion 18a of said axial tubular structure 18 forms or comprises the nozzle element 5 of the aerosol-generating system 4, 5, 6, whereas the downward portion 18b of said axial tubular structure 18 constitutes an upstream connecting-portion 17 for connecting a gas line thereto, preferably pressurized air (> 1 bar).

Furthermore, an inner sleeve conduit 12 projects axially and upwardly into the liquid tank 11, and forms a sleeve around the nozzle element 5 of the aerosol-generating system 4, 5, 6, i.e. around the upward portion 18a of said axial tubular structure 18.

Preferably, the deflector 4, the inner conduit 8 and the inner sleeve conduit 12 are made in one-piece, preferably by molding or the like of a plastic material or similar.

The inner sleeve conduit 12 is separated from the nozzle element 5 by a spacing 13, such as a very small passage, and comprises an upper outlet 14 facing the nozzle orifice 6 of the nozzle element 5.

The deflector element 4, namely the trapezoidal or triangular-shape portion, is arranged so as to face the nozzle orifice 6 of the nozzle element 5 and the upper outlet 14 of the inner sleeve conduit 12, and is arranged at the exit of the lower end 8a of the inner conduit 8 of the nebulizer body 2.

The lower inlet 15 of the inner sleeve conduit 12 is located very close to the bottom 16 of the liquid tank 11 of the reservoir 3, preferably at a distance of less than about 2 mm, so that the liquid L contained into the tank 11 can circulate between the bottom 16 and the extremity of the inner sleeve conduit 12 so as to penetrate into the lower inlet 15 of the inner sleeve conduit 12.

The role of the axial tubular structure 18, i.e. conduit, is to convey a pressurized gas delivered by a gas source (not shown), such as compressed air, fluidly connected to the downward portion i8b of the axial tubular structure 18. The gas flow travels into the lumen of the axial tubular structure 18 and is delivered by the nozzle orifice 6 of the nozzle element 5 for creating the mist or aerosol as explained below.

Preferably, the reservoir 3 is detachably attached to the nebulizer body 2, for instance they are snap-fitted or threadedly-coupled together, or similarly adapted for repeated attachment/detachment.

When the reservoir 3 is attached to the nebulizer body 2, at least a part of the nebulization chamber 7 is positioned between the peripheral wall 2a of the nebulizer body 2 and the axially-arranged inner conduit 8, and is further located above the liquid tank 11.

For generating the aerosol mist, the liquid contained into the tank 11 of the reservoir 3 should be able to circulate in the spacing 13 for reaching the aerosol-generating system 4, 5, 6, that can generate the aerosol mist thanks to a Venturi effect or suction effect. This is well-known in the art.

In a few words, pressurized air is conveyed (see arrow B in Fig. 3) and delivered by the inner conduit 18 of reservoir 3, at a high speed and a constant flowrate, which creates a suction effect that moves the liquid solution out of the tank 11.

The liquid solution travels in the spacing 13, i.e. it is upwardly pulled by the suction effect, and is then delivered by the upper outlet 14, e.g. a small-diameter orifice (e.g., < 5 mm in diameter), of the inner sleeve conduit 12 toward the deflector 4. When the liquid enters into contact with the deflector 4, a mist of liquid droplets is created by the impact of the liquid on the deflector element 4. The smaller droplets thus obtained create the desired aerosol mist that is recovered into the nebulization chamber 5. The aerosol mist can thereafter be inhaled by the patient via the respiratory interface 22, namely a mouthpiece, or a mouth or facial respiratory mask.

The choice of the most suitable interface 22 depends on the type of medication to be taken and on the type of patients i.e. the facial mask is suitable for non-collaborative pediatric patients, while the mouthpiece and mouth mask are better for adults. In the case of a mouthpiece, the distal end of the mouthpiece is designed so as to form a "beak-shape nozzle" that the patient takes in mouth, whereas in the case of a mouth or facial respiratory mask, the distal end is designed so as to form an enclosure that is large enough for receiving at least a part of the patient's nose or nose and mouth.

Further, as above mentioned, a detachable cover 23 forms the "top" of the nebulizer 1, i.e. the ceiling of the nebulizer body 2.

Said cover 23 comprises a pair of valves 24, 25, namely a one-way inspiration valve 24 allowing ambient air entering into the nebulization chamber 7 via the axially-arranged inner conduit 8 of the nebulizer body 2, during the inspiration phases of the patient, and a one-way expiration valve 25 allowing gas exiting the nebulization chamber 7, during the expiration phases of the patient, especially expired gases that contains CO₂.

The inspiration valve 24 is located so as to face and be in fluid communication with the air inlet of the axially-arranged inner conduit 8, and opens only during inspiration phases of the patient by action of the negative pressure generated by the patient's inspiration, i.e. during inspiration phases. The inspiration valve 24 provides an additional flow of ambient air that travels into inner conduit 8 (see arrow A in Fig. 3) and then enters into the nebulization chamber 7 thereby "boosting" the nebulization (due to a "double" Venturi effect), significantly increasing the amount of aerosol mist formed into said nebulizing chamber 7, and further increasing the nebulization rate of the liquid drug, i.e. the solution containing the medication.

In contrast, the expiration valve 25 opens by action of the CO₂-containing gases exhaled by the patient that create an overpressure (i.e. pressure > 1 bar) in the respiratory interface 22 and at the inlet of the nebulization chamber 7, while the patient is exhaling gases. Said overpressure allows gases to be vented from the nebulizer 1, through the expiration valve 25, when the patient exhales into the respiratory interface 22. In other words, the one-way expiration valve 25 allows said CO₂-containing gas to be vented to the atmosphere.

All the components of the nebulizer 1, i.e. cover, nebulizer body 2, reservoir 3..., are preferably made of plastic material(s), such as PP, PC, ABS, PA, PSU or similar polymer materials, while the patient interface 22 are made of a soft elastomeric material, such as silicone or the like.

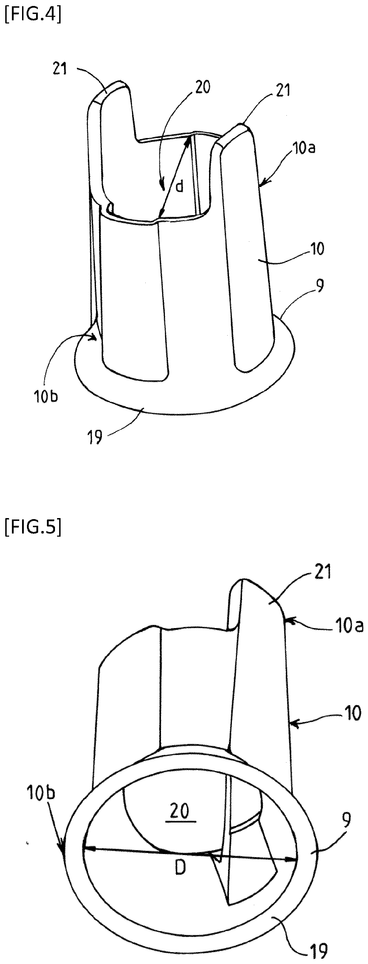

According to the present invention, the lower end 8a of the axially-arranged inner conduit 8 comprises a hollow tubular element 10 (Fig. 4 & 5) comprising a first end 10a and a second end 10b, and a flared-out portion 9. Said flared-out portion 9 forms a kind of skirt or the like that projects outwardly into reservoir 3.

The tubular element 10 (Fig. 4 & 5) is detachably attached to the lower end 8a of the inner conduit 8, as shown in Fig. 2 & 3, by its first free end 10a.

More precisely, the tubular element 10 comprises, at its second free end 10b, the flared-out portion 9. It forms a sleeve or a kind of "bell" around the lower end 8a of the inner conduit 8 and is further arranged around the nozzle element 5 comprising the nozzle orifice 6.

With such an arrangement, the aerosol mist created by the aerosol-generating system 4, 5, 6 is mainly sent to the nebulization chamber 7, and the proportion of aerosol mist that condenses on the inner parts of the nebulizer body 2 and is hence wasted, is limited/minimized. Indeed, the flared-out portion 9, i.e. the flared-out skirt that projects into the tank 11 of reservoir 3, condenses a part of the mist thereby forming droplets that can return to the liquid tank 11 in lieu of being wasted due to their condensation on the inner walls of the nebulizer.

Comparative tests have been made with the nebulizer 1 of the present invention equipped with a tubular element 10 (Fig. 4 & 5) with a flared-out portion 9 as shown in Fig. 2 & 3, and with a prior art nebulizer having a similar structure but without such a flared-out portion.

The tanks of both nebulizers were filed with 3 cc of a liquid solution that is nebulized/aerosolized in the same way, i.e. using compressed air for creating the Venturi effect as above explained.

Once the tanks were empty, the amount of liquid condensed/wasted on the inner walls of both nebulizers has been evaluated/measured. The results are given in the following Table.

**Table**

| Nebulizer | Present invention | Prior art |
|---|---|---|
| Amount of liquid in the tank prior to nebulization (in cc=cm³) | 3 | 3 |
| Amount of liquid wasted on inner parts after nebulization (in cc) | 0,8 | 1,3 |
| Proportion of wasted liquid | 26.7% | 43,3% |

As one can see, the quantity of liquid lost is only of 26,7% with the nebulizer 1 of the present invention, whereas it is of 43,3% with a nebulizer that does not comprise any flared-out portion according to the present invention.

This is an important improvement as, with the nebulizer 1 of the present invention, about 17% of aerosolized drug solution is available for treating the patient in lieu of being lost on the inner walls of the nebulizer. This is due to the fact that the flared-out portion 9 acts as a liquid condenser that retains a part of the aerosolized mist allowing it to condense on the inner surface of said flared-out portion 9 and to fall back, by gravity, into the tank 11 of the reservoir 3 in lieu of being wasted on the inner walls of the nebulizer body 2.

Figures 4 and 5 show an embodiment of a hollow tubular element 10 with a flared-out portion 9 forming a skirt projecting outwardly that can be arranged in a nebulizer 1 according to the present invention. Said tubular element 10 forms a sleeve or similar around the downstream end or lower end 8a of the inner conduit 8, as shown in Fig. 2 & 3.

The hollow tubular element 10 further comprises an inner lumen 20 configured and sized so as to match the peripheral contours, i.e. outer wall, of the lower end 8a of the inner conduit 8. Preferably, the tubular element 10 has a generally cylindrical shape with an enlarged-diameter end forming the flared-out portion 9. Said flared-out portion 9 comprises an annular border 19 located at its extremity.

Further, the tubular element 10 also comprises elongated parts 21, such as elongated arms, projecting upwardly, for instance two (or more) parallel elongated arms 21 as shown in Fig. 4 & 5, that are used for maintaining the tubular element 10 in secured position around the lower end 8a of the inner conduit 8 and integral with the nebulizer body 2.

The elongated arms 21 are designed to cooperate with internal elongated lodgings 26 arranged in the nebulizer body 2 for thereby fixing said tubular element 10, i.e. securing the tubular element 10 axially around the inner conduit 8. Preferably, the elongated arms 21 are plugged or the like into the internal elongated lodgings 26.

Further, the tubular element 10 is preferably molded in one-piece and made of a plastic material; however, it could be also realized in sub-units assembled together.

The flared-out portion 9 comprises an enlarged maximum diameter D (at the annular border 19) that is greater than the inner maximum diameter d of the hollow tubular element 10 as shown in Fig. 4 and 5. For instance, the flared-out portion 9 comprises an enlarged diameter D of less than 3 cm, preferably of between 2 and 3 cm, whereas the inner diameter d of hollow tubular element 10 is greater than 1 cm and less than 2.5 cm, preferably of between about 1,5 and about 2 cm.

Furthermore, the tubular element 10 also comprises one or several guiding structures, such as two longitudinal parallel grooves as shown in Fig. 4 & 5 or the like, cooperating with one or several fingers, picots or any other complementary structure arranged in the nebulizer body 2 for well-positioning the hollow tubular element 10 around the axially-arranged inner conduit 8.

The nebulizer 1 of the present invention is usable in combination with a source of gas, such as compressed air, for nebulizing a drug-containing solution and thereby obtaining a mist containing small droplets of medication having suitable sizes so as to be able to reach the lower airways of the patient that has to inhale said mist in the frame of his/her aerosoltherapy.

## Claims

1. Nebulizer (1) for aerosolizing a liquid, the nebulizer comprising:
- a reservoir (3) for receiving the liquid (L) to be aerosolized,
- a nebulizer body (2) comprising an aerosol-generating system (4; 5, 6) for generating an aerosol and a nebulization chamber (7) for containing the aerosol, and
- a detachable cover (23) detachably fixed to the nebulizer body (2), said detachable cover (23) forming the ceiling of the nebulizer body (2),
and wherein :
- the aerosol-generating system (4, 5, 6) comprises a nozzle element (5) comprising a nozzle orifice (6) and a deflector element (4) facing the nozzle orifice (6) of the nozzle element (5),
- the nebulizer body (2) comprises an axially-arranged inner conduit (8) comprising a lower end (8a),
- the nebulization chamber (7) is arranged around at least a part of said axially-arranged inner conduit (8), and
- a hollow tubular element (10) comprising a flared-out portion (9) is arranged around the lower end (8a) of the axially-arranged inner conduit (8), said flared-out portion (9) forming a skirt projecting outwardly into the reservoir (3), said flared-out portion (9) comprising an enlarged diameter (D) that is greater than the inner diameter (d) of the hollow tubular element (10), said flared-out portion (9) being arranged around the nozzle element (5) comprising the nozzle orifice (6), said tubular element (10) further comprising a first end (10a) and a second end (10b), said tubular element (10) being detachably attached to the axially-arranged inner conduit (8) by the first end (10a) and comprises the flared-out portion (9) at the second end (10b), and
- the detachable cover (23) comprises a one-way inspiration valve (24) and a one-way expiration valve (25) arranged on said detachable cover (23) for controlling air flows entering into or exiting the nebulizer (1),
and wherein :
- the inspiration valve (24) is located so as to face and be in fluid communication with an air inlet of the axially-arranged inner conduit (8), and is structured so that, in use, it opens only during inspiration phases of the patient, thereby providing an additional flow of air that travels into the inner conduit (8) and then enters into the nebulization chamber (7) thereby "boosting" the nebulization, and
- the expiration valve (25) is structured so that, in use, it opens by action of the gases exhaled by the patient.

2. Nebulizer according to the preceding Claim, **characterized in that** the flared-out portion (9) comprises an enlarged diameter (D) of less than 3 cm.

3. Nebulizer according to any one of the preceding Claims, **characterized in that** the inner diameter (d) of the hollow tubular element (10) is greater than 1 cm and less than 2.5 cm.

4. Nebulizer according to any one of the preceding Claims, **characterized in that** the tubular element (10) comprising the flared-out portion (9) is arranged so as to form a sleeve around the lower end (8a) of the axially-arranged inner conduit (8).

5. Nebulizer according to Claim 4, **characterized in that** the tubular element (10) is co-axially arranged around the inner conduit (8).

6. Nebulizer according to any one of the preceding Claims, **characterized in that** the tubular element (10) comprises at the first end (10a) one or several elongated parts (21) projecting upwardly cooperating with one or several lodgings (26) for securing the tubular element (10) into the nebulizer body (2).

7. Nebulizer according to any one of the preceding Claims, **characterized in that** the reservoir (3) comprises:
- a liquid tank (11) for receiving the liquid (L) to be aerosolized, and
- an inner sleeve conduit (12) projecting axially and upwardly into the liquid tank (11), said inner sleeve conduit (12) forming a sleeve around the nozzle element (5) of the aerosol-generating system (4; 5, 6), said inner sleeve conduit (12) being separated from the nozzle element (5) by a spacing (13) and comprising an upper outlet (14) facing the nozzle orifice (6) of the nozzle element (5).

8. Nebulizer according to Claim 7, **characterized in that** the deflector element (4) is facing the nozzle orifice (6) of the nozzle element (5) and the upper outlet (14) of the inner sleeve conduit (12), and is arranged at the exit of the lower end (8a) of the axially-arranged inner conduit (8).

9. Nebulizer according to any one of Claims 7 or 8, **characterized in that** the inner sleeve conduit (12) further comprises a lower inlet (15) located close to the bottom (16) of the liquid tank (11) of the reservoir (3).

10. Nebulizer according to Claims 7 to 9, **characterized in that** the deflector (4), the axially-arranged inner conduit (8) and the inner sleeve conduit (12) are made in one-piece.

11. Nebulizer according to any one of the preceding Claims, **characterized in that** it further comprises a respiratory interface (22) in fluid communication with the nebulization chamber (7).

12. Nebulizer according to Claim 11, **characterized in that** the respiratory interface (22) comprises a mouthpiece, a mouth mask or a facial mask.

## Patentansprüche

1. Vernebler (1) zur Aerosolisierung einer Flüssigkeit, wobei der Vernebler folgendes umfasst:
- einen Behälter (3) zur Aufnahme der Flüssigkeit (L), die aerosoliert werden soll,
- einen Verneblerkörper (2), der ein aerosolerzeugendes System (4; 5, 6) zum Erzeugen eines Aerosols und eine Vernebelungskammer (7) zur Aufnahme des Aerosols umfasst, und
- eine abnehmbare Abdeckung (23), die abnehmbar an dem Verneblerkörper (2) angebracht ist, wobei die abnehmbare Abdeckung (23) die Decke des Verneblerkörpers (2) bildet,
und wobei:
- das aerosolerzeugende System (4, 5, 6) ein Düsenelement (5) umfasst, das eine Düsenöffnung (6) und ein der Düsenöffnung (6) des Düsenelements (5) zugewandtes Deflektorelement (4) umfasst,
- der Verneblerkörper (2) eine axial angeordnete interne Leitung (8) umfasst, die ein unteres Ende (8a) umfasst,
- die Vernebelungskammer (7) um mindestens einen Teil der axial angeordneten internen Leitung (8) herum angeordnet ist, und
- ein hohles rohrförmiges Element (10), das einen aufgeweiteten Abschnitt (9) umfasst, um das untere Ende (8a) der axial angeordneten internen Leitung (8) herum angeordnet ist, wobei der aufgeweitete Abschnitt (9) eine nach außen in den Behälter (3) ragende Einfassung bildet, wobei der aufgeweitete Abschnitt (9) einen vergrößerten Durchmesser (D) umfasst, der größer ist als der Innendurchmesser (d) des hohlen rohrförmigen Elements (10), wobei der aufgeweitete Abschnitt (9) um das Düsenelement (5) herum angeordnet ist, das die Düsenöffnung (6) umfasst, wobei das rohrförmige Element (10) weiter ein erstes Ende (10a) und ein zweites Ende (10b) umfasst, wobei das rohrförmige Element (10) durch das erste Ende (10a) abnehmbar an der axial angeordneten internen Leitung (8) angebracht ist und an dem zweiten Ende (10b) den aufgeweiteten Abschnitt (9) umfasst, und
- die abnehmbare Abdeckung (23) ein Einweg-Einatmungsventil (24) und ein Einweg-Ausatmungsventil (25) umfasst, die auf der abnehmbaren Abdeckung (23) angeordnet sind, um Luftströme zu steuern, die in den Vernebler (1) eintreten oder aus diesem austreten,
und wobei:
- das Einatmungsventil (24) so angeordnet ist, dass es einem Lufteinlass der axial angeordneten internen Leitung (8) zugewandt ist und mit diesem in Fluidverbindung steht, und so strukturiert ist, dass es sich bei Verwendung nur während der Einatmungsphasen des Patienten öffnet, wodurch es einen zusätzlichen Luftstrom bereitstellt, der in die interne Leitung (8) strömt und dann in die Vernebelungskammer (7) eintritt, wodurch er die Vernebelung "boostet", und
- das Ausatmungsventil (25) so strukturiert ist, dass es sich bei Verwendung durch die Wirkung der von dem Patienten ausgeatmeten Gase öffnet.

2. Vernebler nach dem vorstehenden Anspruch, **dadurch gekennzeichnet, dass** der aufgeweitete Abschnitt (9) einen vergrößerten Durchmesser (D) von weniger als 3 cm umfasst.

3. Vernebler nach einem der vorstehenden Ansprüche, **dadurch gekennzeichnet, dass** der Innendurchmesser (d) des hohlen rohrförmigen Elements (10) größer als 1 cm und kleiner als 2,5 cm ist.

4. Vernebler nach einem der vorstehenden Ansprüche, **dadurch gekennzeichnet, dass** das rohrförmige Element (10), das den aufgeweiteten Abschnitt (9) umfasst, so angeordnet ist, dass es eine Hülse um das untere Ende (8a) der axial angeordneten internen Leitung (8) bildet.

5. Vernebler nach Anspruch 4, **dadurch gekennzeichnet, dass** das rohrförmige Element (10) koaxial um die interne Leitung (8) herum angeordnet ist.

6. Vernebler nach einem der vorstehenden Ansprüche, **dadurch gekennzeichnet, dass** das rohrförmige Element (10) an dem ersten Ende (10a) ein oder mehrere nach oben ragende längliche Teile (21) umfasst, die mit einer oder mehreren Aufnahmen (26) zusammenwirken, um das rohrförmige Element (10) im Verneblerkörper (2) zu befestigen.

7. Vernebler nach einem der vorstehenden Ansprüche, **dadurch gekennzeichnet, dass** der Behälter (3) folgendes umfasst:
- einen Flüssigkeitsbehälter (11) zum Aufnehmen der zu aerosolierenden Flüssigkeit (L), und
- eine interne Hülsenleitung (12), die axial und nach oben in den Flüssigkeitsbehälter (11) hineinragt, wobei die interne Hülsenleitung (12) eine Hülse um das Düsenelement (5) des aerosolerzeugenden Systems (4; 5, 6) bildet, wobei die interne Hülsenleitung (12) von dem Düsenelement (5) durch einen Abstand (13) getrennt ist und einen oberen Auslass (14) umfasst, der der Düsenöffnung (6) des Düsenelements (5) zugewandt ist.

8. Vernebler nach Anspruch 7, **dadurch gekennzeichnet, dass** das Deflektorelement (4) der Düsenöffnung (6) des Düsenelements (5) und dem oberen Auslass (14) der internen Hülsenleitung (12) zugewandt ist und an dem Ausgang des unteren Endes (8a) der axial angeordneten internen Leitung (8) angeordnet ist.

9. Vernebler nach einem der Ansprüche 7 oder 8, **dadurch gekennzeichnet, dass** die interne Hülsenleitung (12) weiter einen unteren Einlass (15) umfasst, der nahe dem Boden (16) des Flüssigkeitsbehälters (11) des Behälters (3) angeordnet ist.

10. Vernebler nach den Ansprüchen 7 bis 9, **dadurch gekennzeichnet, dass** der Deflektor (4), die axial angeordnete interne Leitung (8) und die interne Hülsenleitung (12) aus einem Stück hergestellt sind.

11. Vernebler nach einem der vorstehenden Ansprüche, **dadurch gekennzeichnet, dass** er weiter eine respiratorische Schnittstelle (22) in Fluidverbindung mit der Vernebelungskammer (7) aufweist.

12. Vernebler nach Anspruch 11, **dadurch gekennzeichnet, dass** die respiratorische Schnittstelle (22) ein Mundstück, eine Mundmaske oder eine Gesichtsmaske umfasst.

## Revendications

1. Nébuliseur (1) pour pulvériser un liquide, le nébuliseur comprenant :
- un réservoir (3) pour recevoir le liquide (L) à pulvériser,
- un corps de nébuliseur (2) comprenant un système de production d'aérosol (4 ; 5, 6) pour produire un aérosol et une chambre de nébulisation (7) pour contenir l'aérosol, et
- un couvercle détachable (23) fixé de façon amovible au corps de nébuliseur (2), ledit couvercle détachable (23) formant le plafond du corps de nébuliseur (2),
et dans lequel :
- le système de production d'aérosol (4, 5, 6) comprend un élément formant buse (5) comprenant un orifice de buse (6) et un élément formant déflecteur (4) faisant face à l'orifice de buse (6) de l'élément formant buse (5),
- le corps de nébuliseur (2) comprend un conduit intérieur (8) agencé de façon axiale comprenant une extrémité inférieure (8a),
- la chambre de nébulisation (7) est agencée autour d'au moins une partie dudit conduit intérieur (8) agencé de façon axiale, et
- un élément tubulaire creux (10) comprenant une portion évasée (9) est agencé autour de l'extrémité inférieure (8a) du conduit intérieur (8) agencé de façon axiale, ladite portion évasée (9) formant une jupe en saillie vers l'extérieur dans le réservoir (3), ladite portion évasée (9) comprenant un diamètre agrandi (D) qui est plus grand que le diamètre intérieur (d) de l'élément tubulaire creux (10), ladite portion évasée (9) étant agencée autour de l'élément formant buse (5) comprenant l'orifice de buse (6), ledit élément tubulaire (10) comprenant en outre une première extrémité (10a) et une seconde extrémité (10b), ledit élément tubulaire (10) étant attaché de façon amovible au conduit intérieur (8) agencé de façon axiale par la première extrémité (10a) et comprend la portion évasée (9) au niveau de la seconde extrémité (10b), et
- le couvercle détachable (23) comprend une valve d'inspiration à sens unique (24) et une valve d'expiration à sens unique (25) agencées sur ledit couvercle détachable (23) pour commander les flux d'air entrant ou sortant du nébuliseur (1),
et dans lequel :
- la valve d'inspiration (24) est située de façon à faire face et être en communication à fluide avec une admission d'air du conduit intérieur (8) agencé de façon axiale et est structurée de sorte que, lors de l'utilisation, elle s'ouvre seulement pendant les phases d'inspiration du patient, fournissant de ce fait un flux supplémentaire d'air qui voyage dans le conduit intérieur (8) et qui entre ensuite dans la chambre de nébulisation (7) « augmentant » de ce fait la nébulisation, et
- la valve d'expiration (25) est structurée de sorte que, lors de l'utilisation, elle s'ouvre par l'action des gaz exhalés par le patient.

2. Nébuliseur selon la revendication précédente, **caractérisé en ce que** la portion évasée (9) comprend un diamètre agrandi (D) de moins de 3 cm.

3. Nébuliseur selon l'une quelconque des revendications précédentes, **caractérisé en ce que** le diamètre intérieur (d) de l'élément tubulaire creux (10) est plus grand que 1 cm et inférieur à 2,5 cm.

4. Nébuliseur selon l'une quelconque des revendications précédentes, **caractérisé en ce que** l'élément tubulaire (10) comprenant la portion évasée (9) est agencé de façon à former un manchon autour de l'extrémité inférieure (8a) du conduit intérieur (8) agencé de façon axiale.

5. Nébuliseur selon la revendication 4, **caractérisé en ce que** l'élément tubulaire (10) est agencé de façon coaxiale autour du conduit intérieur (8).

6. Nébuliseur selon l'une quelconque des revendications précédentes, **caractérisé en ce que** l'élément tubulaire (10) comprend au niveau de la première extrémité (10a) une ou plusieurs parties allongées (21) en saillie vers le haut coopérant avec un ou plusieurs logements (26) pour sécuriser l'élément tubulaire (10) dans le corps de nébuliseur (2).

7. Nébuliseur selon l'une quelconque des revendications précédentes, **caractérisé en ce que** le réservoir (3) comprend :
- un réservoir de liquide (11) pour recevoir le liquide (L) à pulvériser, et
- un conduit formant manchon intérieur (12) en saillie de façon axiale et vers le haut dans le réservoir de liquide (11), ledit conduit formant manchon intérieur (12) formant un manchon autour de l'élément formant buse (5) du système de production d'aérosol (4; 5, 6), ledit conduit formant manchon intérieur (12) étant séparé de l'élément formant buse (5) par un espacement (13) et comprenant une sortie supérieure (14) faisant face à l'orifice de buse (6) de l'élément formant buse (5).

8. Nébuliseur selon la revendication 7, **caractérisé en ce que** l'élément formant déflecteur (4) fait face à l'orifice de buse (6) de l'élément formant buse (5) et au débouché supérieur (14) du conduit formant manchon intérieur (12), et est agencé au niveau de la sortie de l'extrémité inférieure (8a) du conduit intérieur (8) agencé de façon axiale.

9. Nébuliseur selon l'une quelconque des revendications 7 ou 8, **caractérisé en ce que** le conduit formant manchon intérieur (12) comprend en outre une admission inférieure (15) située près du fond (16) du réservoir de liquide (11) du réservoir (3).

10. Nébuliseur selon les revendications 7 à 9, **caractérisé en ce que** le déflecteur (4), le conduit intérieur (8) agencé de façon axiale et le conduit formant manchon intérieur (12) sont faits d'un seul tenant.

11. Nébuliseur selon l'une quelconque des revendications précédentes, **caractérisé en ce qu'**il comprend en outre une interface respiratoire (22) en communication à fluide avec la chambre de nébulisation (7).

12. Nébuliseur selon la revendication 11, **caractérisé en ce que** l'interface respiratoire (22) comprend une embouchure, un masque buccal ou un masque facial.
